(19) Europäisches Patentamt
European Patent Office
Office européen des brevets

(11) Publication number: 0 137 083
B1

(12) **EUROPEAN PATENT SPECIFICATION**

(45) Date of publication of patent specification: **24.06.87**

(51) Int. Cl.$^4$: **C 07 D 281/10,** A 61 K 31/55 // C07C149/42, C07D279/16, C07D277/68

(21) Application number: **83306147.6**

(22) Date of filing: **11.10.83**

(54) **1,5-Benzothiazepine derivatives and production thereof.**

(43) Date of publication of application:
**17.04.85 Bulletin 85/16**

(45) Publication of the grant of the patent:
**24.06.87 Bulletin 87/26**

(84) Designated Contracting States:
**CH DE FR GB LI**

(56) References cited:
**US-A-3 562 257**

**JOURNAL OF THE CHEMICAL SOCIETY, CHEMICAL COMMUNICATIONS, no. 8, published 15th April 1983, pages 450-451, London, GB Y. MAKI et al.: "A simple ring-expansion of 1,4-benzothiazines to give 1,5-benzothiazepines"**

(73) Proprietor: **HAMARI YAKUHIN KOGYO KABUSHIKI KAISHA also known as HAMARI CHEMICALS LTD.**
**4-29, Kunijima 1-chome Higashi-yodogawa-ku**
**Osaka 533 (JP)**

(72) Inventor: **Maki, Yoshifumi**
**22-4, Mitahora-higashi 3-chome**
**Gifu 502 (JP)**
Inventor: **Sako, Magoichi**
**33, Harusato-cho 4-chome Seki**
**Gifu 501-32 (JP)**
Inventor: **Mitsumori, Naomichi**
**112-6, Asahitani Katsuhara-ku**
**Himeji Hyogo 671-12 (JP)**
Inventor: **Maeda, Sadayuki**
**3-19, Kohama 3-chome Suminoe-ku**
**Osaka 559 (JP)**
Inventor: **Takaya, Masahiro**
**121-11, Yukihata-sannotsubo Oaza Yasu-cho**
**Yasu-gun Shiga 520-23 (JP)**

(74) Representative: **Burford, Anthony Frederick et al**
**W.H. Beck, Greener & Co. 7 Stone Buildings**
**Lincoln's Inn**
**London WC2A 3SZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 137 083 B1

**Description**

This invention relates to benzothiazepine derivatives and a method for producing the same. More particularly, the invention relates to 2-phenyl-(or substituted phenyl-)2,3-dihydro-5-substituted-1,5-benzothiazepine-3,4(5H)-diones which can be represented by the following formula (I):

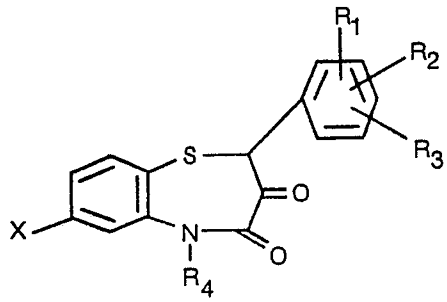

(I)

wherein $R_1$, $R_2$ and $R_3$ each denotes hydrogen, a halogen, a $C_1$—$C_5$ alkyl group, a $C_1$—$C_5$ alkoxy group or a hydroxy group; $R_4$ denotes a $C_1$—$C_5$ alkyl group, an allyl group a $C_1$—$C_5$ alkoxy $C_1$—$C_5$ alkyl group, a $C_1$—$C_5$ alkyl group substituted with a hydroxy group or a halogen, a mono- or di-$C_1$—$C_5$ alkylamino $C_1$—$C_5$ alkyl group or a morpholino $C_1$—$C_5$ alkyl group; X denotes a halogen or hydrogen, and acid salts thereof.

Preferred alkyl groups are those having up to 4 carbon atoms.

Benzothiazepine derivatives are very interesting compounds, because it is known that some of them have several pharmacological activities.

The compounds (I) of this invention have excellent pharmacological effects such as analgesic, antipyretic or antiarrhythmic activities and are useful as medicines. Except for those compounds (1) in which $R_4$ is alkyl when X is hydrogen (which are described in J.C.S. 1983, p. 450—1), the compounds (1) are novel.

The compounds (I) may be produced, for example, by the following synthesis route:

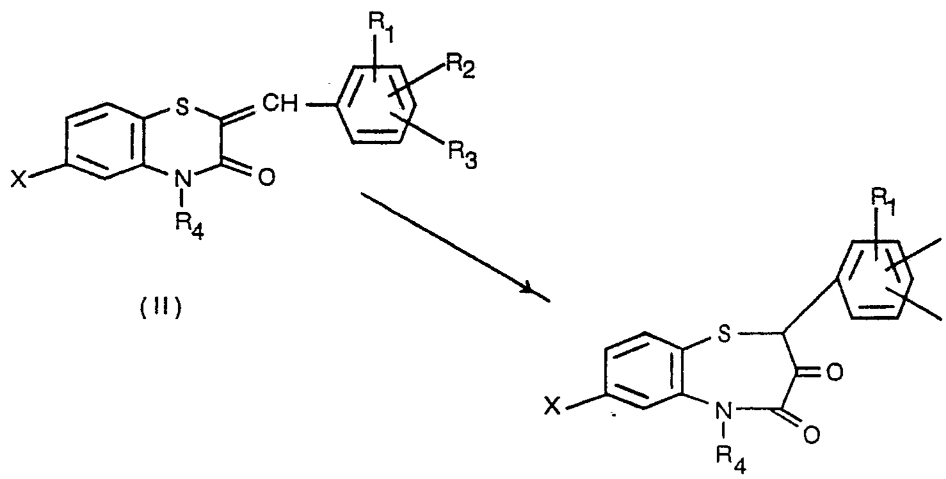

(I)

wherein $R_1$, $R_2$, $R_3$, $R_4$ and X have the same meaning as above.

As illustrated in the above schema, the benzothiazine ring of the compound (II) expands to a benzothiazepine ring by reacting 2-phenyl-(or substituted phenyl-) methylidene-4-substituted-2H-1,4-benzothiazine-3(4H)-one (II) with a trimethylhalosilane, hydrogen peroxide and water, thereby the compound (I) is produced in a high yield. The reaction can be carried out in a non-reactive solvent such as methylene chloride or chloroform under cooling or at room temperature and it proceeds to completion in a period from scores of minutes to several hours.

From the resulting reaction mixture, the compound (I) may be purified by conventional procedure, for example, extraction with a solvent, recrystallization, column chromatography and treatment with activated carbon.

The starting compound (II) can easily be synthesized according to known methods described in V. Baligh and T. Rangarajan, J. Chem. Soc., 1980, 4703; J. Krapcho and C. F. Turk, J. Med. Chem., 1973, 16, 776. Among the compounds (I) thus obtained, a compound having a nitrogen-containing substituent at the 5-position of the benzothiazepine ring can be converted, if desired, to an inorganic acid salt such as hydrochloride, hydrobromide, sulfate, or nitrate, or an organic acid salt such as methanesulfonate, succinate, maleate, or tartrate, by known methods. The ring-expansion of a heterocyclic compound is

known in very rare cases, and there is no report from other inventor(s) so far of a ring-expansion from the six membered ring of benzothiazine to the seven membered ring of benzothiazepine having two carbonyl groups. The ring-expansion of the invention is useful for production of a medicine or an intermediate thereof, because it can be applied to the production of other benzothiazepine derivatives.

The compound (I) of this invention can be employed as an analgesic, antipyretic or antiarrhythmic agent, which may be administered at a daily dose level of 30 to 2,000 mg per kilogram body weight in 3 to 4 installments daily. It may be administerd orally or parenterally in varied dosage form such as powder, granules, tablets, capsules, injection, suppository and cartilage.

In the case of powder, granules, tablets or capsules, the compound (I) may be used, for example, in admixture with pharmacologically acceptable excipients such as starch, lactose, sucrose and calcium carbonate; binders such as starch, carboxymethylcellulose and crystalline cellulose; and lubricants such as talc.

Where the compound (I) is used as an injection, the carrier may, for exmaple, be distilled water or physiological saline. Some pharmacological data and production procedure of the compouds (I) are described in the following Experiment and Examples:

Experiment

Pharmacology of 2-(4-methoxyphenyl)-2,3-dihydro-5-(2-dimethylaminoethyl)-1,5-benzothiazepine-3,4(5H)-dione (Ia) hydrochloride

(1) Minimum Lethal Dose Test in Mice

Groups of 2 mice each, weighing 29—36 g, were treated intraperitoneally with the compound and observed for signs of central, autaonomic and toxic responses for 5 hours. Mortality counts were made on days 0, 1 and 2 (day 0 is the dosing day). The results are shown in Table I.

TABLE 1
Minimum Lethal Dose Test in Mice
— Mortality and Minimum Acute Intraperitoneal Lethal Dose —

| Route | Compound | Dose (mg/kg) | No. of Animals | No. of dead Animals — Day of test 0 | 1 | 2 | Total | Minimum Acute Lethal Dose (mg/kg) |
|---|---|---|---|---|---|---|---|---|
| I.P. | Vehicle | 0 | 2 | 0 | 0 | 0 | 0 | — |
| | Ia | 10 | 2 | 0 | 0 | 0 | 0 | |
| | | 30 | 2 | 0 | 0 | 0 | 0 | 300 |
| | | 100 | 2 | 0 | 0 | 0 | 0 | |
| | | 300 | 2 | 2 | — | — | 2 | |

(2) Analgestic Activity (Mouse Anti-Writhing Method)

Groups of 5 mice each, weighing 26—32 g, were treated intraperitoneally with the compounds. The method is based on the antagonism of a writhing syndrome (abnormal contraction and twisting of the body) produced by the intraperitoneal injection of 10 ml/kg of 0.7% acetic acid-saline solution. Ten minutes after the acetic acid injection, the number of writhes observed in a 10 minute counting period was recorded. The compound was administered 45 minutes before the acetic acid injection. The results are shown in Table II.

TABLE II
Mouse Anti-Writhing Method

| Route | Compound | Dose (mg/kg) | No. of Animals | No. of Writhes (Mean + S.D.) | % Change from Control |
|---|---|---|---|---|---|
| I.P. | Vehicle | 0 | 10 | 10.9 ± 8.9 | — |
| | Ia | 60 | 5 | 0.8 ± 1.3** | −92.7 |
| | Pentazocine | 30 | 5 | 1.8 ± 2.2* | −83.5 |
| | Vehicle | 0 | 5 | 12.8 ± 6.3 | — |
| | Ia | 60 | 5 | 0.0 ± 0.0** | −100.0 |

*: P ≤ 0.05 as compared to the vehicle group
**: P ≤ 0.04 as compared to the vehicle group

(3) Anti-pyretic Activity (Temperature Regulation in Pyretic Rats)

Rats, weighing 220—300 g, were treated subcutaneously in the nape of the neck with 6 ml of 40% suspension of dried Brewer's yeast in distilled water. Groups of 3 rats were treated intraperitoneally with the compound 17 hours challenge. Rectal temperature were recorded with a thermister type thermometer (Type BMA—77) produced by Natsume Seisakusho, Japan) just prior to the drug administration and at 30 minute intervals for 2 hours. The results are shown in Table III.

TABLE III
Effect on Yeast-induced Pyresis in Rats

| Route | Compound | Dose (mg/kg) | No. of Animals | Rectal Temperature (C°, Mean + S.D.) | | | | | | |
|---|---|---|---|---|---|---|---|---|---|---|
| | | | | Time after Treatment (hour) | | | | | Average at 0.5—2.0 Hours Post-treatment | Change C° |
| | | | | 0 | 0.5 | 1.0 | 1.5 | 2.0 | | |
| I.P. | Vehicle | 0 | 3 | 39.6 ± 0.25 | 39.9 ± 0.23 | 39.9 ± 0.15 | 39.7 ± 0.07 | 39.6 ± 0.09 | 39.8 | 0 |
| | Ia | 60 | 3 | 39.7 ± 0.15 | 38.4 ± 0.57 | 38.4 ± 0.35 | 38.8 ± 0.49 | 39.0 ± 0.51 | 38.7 | −1.1 |
| | Aminopyrin | 30 | 3 | 39.6 ± 0.10 | 38.1 ± 0.50 | 37.2 ± 0.58 | 37.2 ± 0.53 | 37.4 ± 0.46 | 37.5 | −2.3 |
| Normal Rat | | | 4 | | | | | 37.3 ± 0.12 | | |

Example 1

To a solution of sodium ethoxide previously prepared from sodium (8.7 g) and absolute ethanol (350 ml), was added 5-chloro-2-benzothiazolinone (70.0 g) with stirring. To this clear solution, methyl iodide (80.3 g) was added dropwise and then the mixture was refluxed for three hours. After removal of the solvent under reduced pressure, the residual oil was treated with water. The resulting precipitates were collected on a filter, washed with water, and air-dried to give 5-chloro-3-methyl-2-benzothiazolinone (73.4 g) as needles. m.p. 106.0—107.5°C.

5-Chloro-3-methyl-2-benzothiazolinone (70.0 g) was added to a mixture of water (118 ml), ethanol (350 ml) and potassium hydroxide (78.6 g). The solution was refluxed with stirring for three hours. After the solvent was evaporated under reduced pressure, the residual oil was dissolved in water. After removal of insoluble yellow crystals by filtration, the filtrate was neutralized with 2N-acetic acid, then resulting oily substance was extracted with ethyl acetate. The extract was washed with water, dried ($Na_2SO_4$), and evaporated to give oily 4-chloro-2-methylaminothiphenol (59.7 g). To a solution of sodium hydroxide (14.0 g) in water (100 ml) was added 4-chloro-2-methylaminothiophenol (59.6 g) with stirring, and then was added dropwise a solution of chloroacetic acid (34.0 g) in water (50 ml) at 20—25°C, followed by heating almost to boiling for three hours.

After the reaction mixture was cooled to room temperature, resulting light yellow precipitates were filtered off, washed with water, and air-dried to give 6-chloro-4-methyl-2H-1,4-benzothiazine-3(4H)-one (68.7 g) as prisms. m.p. 119.0—121.0°C.

To a solution of sodium ethoxide previously prepared from sodium (0.94 g) and absolute ethanol (55 ml), were added 6-chloro-4-methyl-1,4-benzothiazine-3(4H)-one (8 g) and anisaldehyde (5.1 g).

The mixture was then refluxed with stirring for one hour. After cooling, the reaction mixture was diluted with water (25 ml). The resulting precipitated products were collected by suction and recrystallized from methanol to give 6-2(4-methoxyphenylmethylidene)-4-methyl-2H-1,4-benzothiazine-3(4H)-one (4.2 g) as yellow powder. m.p. 136—138°C.

| | | | |
|---|---|---|---|
| Anal. Calcd. for $C_{17}H_{14}NO_2SCl$: | C, 61.54; | H, 4.25; | N, 4.22. |
| Found: | C, 61.35; | H, 4.20; | N, 4.25 |

IR(KBr)$cm^{-1}$: 1626, 1578.

NMR($CDCl_3$)ppm: 3.46 (s, 3H), 3.81 (s, 3H), 6.53—7.67 (br, 7H), 7.77 (s, 1H).

To a stirred solution of 6-chloro-2-(4-methoxyphenylmethylidene)-4-methyl-2H-1,4-benzothiazine-3(4H)-one (1.0 g) in chloroform (30 ml) were added dropwise chlorotrimethylsilane (1.14 g) followed by 30% hydroperoxide (0.54 g) at −5°C. After stirring at −5°C for two hours, water was added and then the stirring was continued for two hours at room temperature. The resulting solution was diluted with chloroform, washed with saturated aqueous sodium bicarbonate, dried ($Na_2SO_4$) and evaporated under reduced pressure.

The residual oil was purified by column chromatography on a silica gel (n-hexane-benzene (1:1)) to give oily 7-chloro-2-(4-methoxyphenyl)-2,3-dihydro-5-methyl-1,5-benzothiazepine-3,4-(5H)-dione (0.65 g).

| | | | |
|---|---|---|---|
| Anal. Calcd. for $C_{17}H_{14}NO_3SCl$: | C, 58.70; | H, 4.06; | N, 4.03. |
| Found: | C, 58.51; | H, 4.08; | N, 3.98. |

IR(Neat)$cm^{-1}$: 1722, 1683, 1604, 1572, 1511.

NMR($CDCl_3$)ppm: 3.44 (s, 3H), 3.76 (s, 3H), 5.41 (s, 1H), 6.65—7.69 (m, 7H).

Example 2

To a solution of sodium ethoxide, previously prepared from sodium (8.7 g) and absolute ethanol (350 ml) was added 5-chloro-2-benzothiazolinone (70.0 g) with stirring. To the clear solution thus obtained, was added dropwise N,N-Dimethylaminoethylchloride (61.0 g), and the mixture was refluxed for three hours.

After removal of the solvent under reduced pressure, the residue was dissolved in ethylacetate, and the solution was washed with aqueous sodium hydroxide. The ethylacetate extract was acidified with dilute hydrochloric acid and the aqueous layer was neutralized with saturated aqueous sodium bicarbonate and then the resulting oily substance was extracted with ethylacetate. The extract was washed with water, dried ($Na_2SO_4$) and evaporated to give 5-chloro-3-(2-dimethylaminoethyl)-2-benzothiazolinone (74.5 g) as prisms. m.p. 62.5—65.5°C. 5-Chloro-3-(2-dimethylaminoethyl)-2-benzothiazolinone (74.5 g) was added to a mixture of water (110 ml), ethanol (350 ml) and potassium hydroxide (75.0 g). The solution was refluxed with stirring for two hours. After removal of the solvent under reduced pressure, the residual oil was dissolved in water, then the aqueous solution was washed with ether. The aqueous layer was washed and neutralized with 2N-acetic acid with weak stirring. The resulting light yellow precipitates were collected on a filter, washed with water and air-dried to give 4-chloro-2-(2-dimethylaminoethylamino)-thiophenol (52.9 g) as prisms. m.p. 126—130°C.

To a solution of sodium hydroxide (9.04 g) in water (130 ml) was added 4-chloro-2-(2-dimethylaminoethylamino)-thiophenol (50.0 g) with stirring.

To the clear solution thus obtained, a solution of chloroacetic acid (20.5 g) in water (30 ml) was added dropwise at a temperature below 20°C, then the mixture was heated to gentle boiling for one hour.

This mixture was cooled to 40°C, and then added with conc. hydrochloric acid (18 ml), slowly.

After refluxing for further four hours, the reaction mixture was made alkaline with aqueous potassium hydroxide and extracted with ethylacetate. The extract washed with water, dried ($Na_2SO_4$) and evaporated to give oily 6-chloro-4-(2-dimethylaminoethyl)-2H-1,4-benzothiazine-3(4H)-one (41.0 g).

To a solution of sodium, ethoxide, previously prepared from sodium (0.76 g) and absolute ethanol (60.0 ml) were added 6-chloro-4-(2-dimethylaminoethyl)-2H-1,4-benzothiazine-3(4H)-one (8.0 g) and anisaldehyde (4.8 g).

The mixture was refluxed with stirring for one hour. After cooling, the reaction mixture was acidified with dilute hydrochloric acid and concentrated under reduced pressure. The resulting oily substance was extracted with chloroform. The extract was washed with a small amount of water, dried ($Na_2SO_4$) and evaporated. The residue soon solidified which was recrystallized from iso-propyl alcohol to give 6-chloro-2-(4-methoxyphenylmethylidene)-4-(2-dimethylaminoethyl)-2H-1,4-benzothiazine-3(4H)-one (4.3 g) hydrochloride as yellow plates. m.p. 196.0—230.5°C.

Anal. Calcd. for $C_{20}H_{21}N_2O_2SCl.HCl$: C, 56.47; H, 5.21; N, 6.59.
Found: C, 56.30; H, 5.23; N, 6.70.
IR(KBr)$cm^{-1}$: 2580, 2425, 1638, 1598, 1578, 1503.
NMR($CDCl_3$)ppm: 2.86 (s, 6H), 3.10—3.60 (br, 2H), 3.80 (s, 3H), 4.20—4.64 (br, 2H), 6.64—7.68 (m, 7H), 7.69 (s, 1H).

To a stirred suspension of 6-chloro-2-(4-methoxyphenylmethylidene)-4-(2-dimethylaminoethyl)-2H-1,4-benzothiazine-3(4H)-one (2.0 g) in chloroform (30 ml) were added dropwise chlorotrimethylsilane (1.79) followed by 30% hydroperoxide (0.83 g) at −5°C. The mixture was stirred for two hours at the same temperature. After water (2.7 ml) was added to the mixture, its temperature was raised to room temperature and then the stirring was continued for another two hours.

The resulting solution was diluted with chloroform, washed with saturated aqueous sodium bicarbonate, dried ($Na_2SO_4$), and evaporated under reduced pressure. The residual oil was purified by column chromatography on silica gel (chloroform-ethanol) (30:1)) to give oily 7-chloro-2-(4-methoxyphenyl)-2,3-dihydro-5-(2-dimethylaminoethyl)-1,5-benzothiazepine-3,4(5H)-dione (1.2 g).

Anal. Calcd. for $C_{20}H_{21}N_2O_3SCl$: C, 59.33; H, 5.23; N, 6.92.
Found: C, 58.85; H, 5.25; N, 6.88.
IR(Neat)$cm^{-1}$: 2825, 2760, 1726, 1665, 1609, 1578, 1511.
NMR($CDCl_3$-$D_2O$)ppm: 2.39 (s, 6H), 2.39—2.75 (br, 2H), 3.74 (s, 3H), 3.83—4.27 (br, 2H), 6.55—7.67 (m, 7H).

In the same manner as described in Example 2, the following compounds were obtained:
7-Chloro-2-phenyl-2,3-dihydro-5-(2-dimethylaminoethyl)-1,5-benzothiazepine-3,4(5H)-dione:

Anal. Calcd. for $C_{19}H_{19}N_2O_2SCl$: C, 60.87; H, 5.11; N, 7.47.
Found: C, 61.03; H, 5.02; N, 7.45.
IR(Neat)$cm^{-1}$: 2810, 2760, 1721, 1660, 1576.
NMR($CDCl_3$-$D_2O$)ppm: 2.22 (s, 6H), 2.41—2.78 (br, 2H), 3.58—4.34 (br, 2H), 6.70—7.74 (m, 8H).

7-Chloro-2-(4-chlorophenyl)-2,3-dihydro-5-(2-dimethylaminoethyl)-1,5-benzothiazepine-3,4(5H)-dione:
Anal. Calcd. for $C_{19}H_{18}N_2O_2SCl_2$: C, 55.75; H, 4.43; N, 6.84.
Found: C, 55.10; H, 4.53; N, 6.77.
IR(Neat)$cm^{-1}$: 2805, 2760, 1718, 1660, 1573.
NMR($CDCl_3$-$D_2O$)ppm: 2.21 (s, 6H), 2.39—2.84 (br, 2H), 3.44—4.28 (br, 2H), 6.50—7.56 (m, 7H).

7-Chloro-2-(3,4-dimethoxyphenyl)-2,3-dihydro-5-(2-dimethylaminoethyl)-1,5-benzothiazepine-3,4(5H)-dione:

Anal. Calcd. for $C_{21}H_{23}N_2O_4SCl$: C, 57.99; H, 5.33; N, 6.44.
Found: C, 58.10; H, 5.28; H, 6.35.
IR(Neat)$cm^{-1}$: 2810, 2760, 1722, 1661, 1578.
NMR($CDCl_3$-$D_2O$)ppm: 2.23 (s, 6H), 2.43—2.83 (br, 2H), 3.40—4.32 (br, 2H), 3.83 (s, 6H), 6.40—7.72 (m, 6H).

7-Chloro-2-(3,4,5-trimethoxyphenyl)-2,3-dihydro-5-(2-dimethylaminoethyl)-1,5-benzothiazepine-3,4(5H)-dione:

Anal. Calcd. for $C_{22}H_{25}N_2O_5SCl$: C, 56.83; H, 5.42; N, 6.02.
Found: C, 56.90; H, 5.40; N, 5.98.
IR(Neat)$cm^{-1}$: 2810, 2760, 1725, 1660, 1575.
NMR($CDCl_3$-$D_2O$)ppm: 2.25 (s, 6H), 2.42—2.81 (br, 2H), 3.37—4.37 (br, 2H, 3.88 (s, 9H), 6.49—7.77 (m, 5H).

7-Chloro-2-(4-methoxyphenyl)-2,3-dihydro-5-(3-dimethylaminopropyl)-1,5-benzothiazepine-3,4(5H)-dione:

Anal. Calcd. for $C_{21}H_{23}N_2O_3SCl$: C, 60.21; H, 5.53; N, 6.69.
Found: C, 60.20; H, 5.49; N, 6.55.
IR(Neat)$cm^{-1}$: 2805, 2755, 1720, 1663, 1603, 1576.
NMR($CDCl_3$-$D_2O$)ppm: 1.43—2.67 (br, 4H), 2.20 (s, 6H), 3.63—4.31 (br, 2H), 3.71 (s, 3H), 6.55—7.75 (m, 7H).

7-Chloro-2-(4-methoxyphenyl)-2,3-dihydro-5-(2-morpholinoethyl)-1,5-benzothiazepine-3,4(5H)-dione:
Anal. Calcd. for $C_{22}H_{23}N_2H_4SCl$: C, 59.12; H, 5.19; N, 6.27.
Found: C, 58.83; H, 5.20; N, 6.22.
IR(Neat)$cm^{-1}$: 2880, 2759, 1720, 1660, 1603, 1574.
NMR($CDCl_3$-$D_2O$)ppm: 2.07—2.87 (br, 6H, 3.15—4.35 (br, 6H), 3.75 (s, 3H), 6.43—7.77 (m, 7H).

Example 3

A solution of 2H-1,4-benzothiazine-3(4H)-one (2 g) and 4-chlorobenzaldehyde (2.6 g) in dimethylformamide (15 ml) was treated with sodium methylate (0.98 g). The temperature of the mixture rose spontaneously. After cooling to room temperature, the reaction mixture was stirred and heated at 110°C with a continuous removal of a low boiling substance for 3 hours. The reaction mixture was cooled to 30°C, and poured onto 100 ml of iced water. The resulting yellow solid was filtered and recrystallized from a mixture of dimethylformamide and isopropyl alcohol. 2-(4-Chlorophenylmethylidene)-2H-1,4-benzothiazine-3(4H)-one (3.3 g) was obtained as yellow crystals. m.p. 245—248°C.

In a smaller manner as described in Example 3, the following compounds were obtained:

2-(4-Methylphenyl)methylidene-2H-1,4-benzothiazine-3(4H)-one, m.p. 266° to 274°C (recrystallized from a mixture of dimethylformamide and isopropyl alcohol);

2-(2,4-Dichlorophenyl-methylidene)-2H-1,4-benzothiazine-3(4H)-ne, m.p. 238° to 240°C.

IR(Nujol)$cm^{-1}$: 3160, 1660, 1580, 1550.
Anal. Calcd. for $C_{15}H_9OSNCl_2$: C, 62.85; H, 3.16; N, 4.78.
Found: C, 62.88; H, 3.08; N, 4.78.

2-(3,4,5-Trimethoxyphenylmethylidene)-2H-1,4-benzothiazine-3(4H)-one, m.p. 204° to 205°C.

Anal. Calcd. for $C_{18}H_{17}O_4SN$: C, 62.97; H, 4.99; N, 4.08.
Found: C, 62.89; H, 5.02; N, 4.18.
IR(Nujol)$cm^{-1}$: 3150, 1650, 1585.

Example 4

2-(4-Chlorophenylmethylidene)-2H-1,4-benzothiazine-3(4H)-one (2.0 g) was reacted with sodium hydroxide (0.72 g) in methyl ethyl ketone (20 ml) at 80°C for 30 minutes. After the solution turned clear, it was cooled to room temperature. N-(2-chloroethyl)morpholine hydrochloride (1.7 g) was added to the mixture. The organic solvent was evaporated under reduced pressure. The resulting yellow solid was filtered and washed with water and with methanol. It was recrystallized from a mixture of dimethylformamide and isopropyl alcohol. 2-(4-Chlorophenyl)-methylidene-4-(2-morpholinoethyl)-2H-1,4-benzothiazine-3(4H)-one (2.0 g) was obtained as yellow crystals, m.p. 173° to 175°C.

Anal. Calcd. for $C_{21}H_{22}O_2SN_2Cl$: C, 62.91; H, 5.28; N, 6.99.
Found: C, 62.85; H, 5.33; N, 7.22.
IR(Nujol)$cm^{-1}$: 1630, 1590, 1575.
NMR($CDCl_3$)ppm: 2.56 (t, 4H), 2.70 (t, 2H), 3.70 (t, 4H), 4.22 (t, 2H), 6.86—7.60 (m, 8H), 7.72 (s, 1H).

In a similar manner as described in Example 4, the following compounds were obtained:

2-(4-Methoxyphenylmethylidene)-4-(2-morpholinoethyl)-2H-1,4-benzothiazine-3(4H)one, m.p. 183° to 184°C (recrystallized from tetrahydrofuran).
Anal. Calcd. for $C_{22}H_{24}O_3SN_2$: C, 66.65; H, 6.10; N, 7.07.
Found: C, 66.55; H, 5.85; N, 7.07.
IR(Nujol)$cm^{-1}$: 1630, 1605, 1590.

NMR($CDCl_3$)ppm: 2.56 (t, 4H), 2.69 (t, 2H), 3.68 (t, 4H), 3.80 (s, 3H), 4.20 (t, 2H), 6.84—7.60; (m, 8H), 7.74 (s, 1H).

2-(4-Methylphenylmethylidene)-4-(2-morpholinoethyl)-2H-1,4-benzothiazine-3(4H)-one, m.p. 119° to 121°C (recrystallized from a mixture of dimethylformamide and isopropyl alcohol).

Anal. Calcd. for $C_{22}H_{24}O_2SN_2$: C, 69.45; H, 6.36; N, 7.36.
Found: C, 69.40; H, 6.33; N, 7.50.
IR(Nujol)$cm^{-1}$: 1640, 1590, 1580, 1560.
NMR($CDCl_3$)ppm: 2.38 (s, 3H), 2.55 (t, 4H), 2.73 (t, 2H), 3.72 (t, 4H, 4.25 (t, 2H), 6.92—7.60 (m, 8H), 7.80 (s, 1H).

2-(2,4-Dichlorophenylmethylidene)-4-(2-morpholinoethyl)-2H-1,4-benzothiazine-3(4H)-one, m.p. 169° to 173°C (recrystallized from a mixture of dimethylformamide and methanol).

Anal. Calcd. for $C_{21}H_{20}O_2SN_2Cl_2$: C, 57.93; H, 4.63; N, 6.43.
Found: C, 58.04; H, 4.55; N, 6.50.
IR(Nujol)$cm^{-1}$: 1645, 1590, 1580.
NMR($CDCl_3$)ppm: 2.36 (t, 4H), 2.70 (t, 2H), 3.68 (t, 4H), 4.22 (t, 2H), 6.82—7.50 (m, 7H), 7.86 (s, 1H).

2-(3,4,5-Trimethoxyphenylmethylidene)-4-(2-morpholinoethyl)-2H-1,4-benzothiazine-3(4H)-one, m.p. 138° to 139°C.

Anal. Calcd. for $C_{24}H_{28}O_5SN_2$: C, 63.14; H, 6.18; N, 6.14.
Found: C, 63.26; H, 6.08; N, 6.22.
IR(Nujol)$cm^{-1}$: 1640, 1595, 1575.
NMR($CDCl_3$)ppm: 2.58 (t, 4HO, 2.76 (t, 2H), 3.74 (t, 4H), 3.88 (s, 3H), 3.90 (s, 3H), 3.92 (s, 3H), 4.26 (t, 2H), 6.68—7.38 (m, 6H), 8.00 (s, 1H).

## Example 5

2-(2,4-Dichlorophenylmethylidene)-2H-1,4-benzothiazine-3(4H)-one (3.0 g) in methyl ethyl ketone (10 ml) was treated with sodium hydroxide (0.98 g) at 80°C for 30 minutes. The reaction mixture was cooled to room temperature and added with dimethylaminoethyl chloride (1.7 g), refluxed for 3 hours and cooled to 30°C. The organic layer was washed with 20 ml of water two times, dried and evaporated under reduced pressure. The residue was dissolved in 20 ml of isopropyl alcohol and added with 0.9 ml of conc. HCl. The resulting precipitates were filtered and recrystallized from 30 ml of isopropyl alcohol to give 2-(2,4-Dichlorophenylmethylidene)-4-(2-dimethylaminoethyl)-2H-1,4-benzothiazine-3(4H)-one hydrochloride as yellow crystals. m.p. 238° to 242°C.

Anal. Calcd. for $C_{19}H_{19}OSN_2Cl_3$: C, 53.10; H, 4.46; N, 6.52.
Found: C, 53.25; H, 4.55; N, 6.40.
IR(Nujol)$cm^{-1}$: 2670, 1635, 1585, 1570, 1560.
NMR(DMSO-$d_6$)ppm: 2.92 (s, 6H), 3.44 (t, 2H), 4.54 (t, 3H), 6.96—7.66 (m, 7H), 7.84 (s, 1H).

## Example 6

2-(3,4,5-Trimethoxyphenylmethylidene)-4-(2-morpholinoethyl)-1,4-benzothiazine-3(4H)-one (0.5 g) was dissolved in 5 ml of chloroform. Chlorotrimethylsilane (1 ml) was added thereto. The mixture was stirred for 30 min. at room temperature and cooled below 0°C. Another 1 ml of trimethylchlorosilane was added and then 30% hydrogen peroxide (0.26 g) was added dropwise for 30 minutes, while the temperature of the mixture maintained below 0°C. Water (1 ml) was added to the solution. The reaction was completed after stirring at room temperature for 2 hours. The organic phase was washed with an aqueous solution of sodium bicarbonate, and with water two times. The organic layer was separated, dried and evaporated under reduced pressure. The residue was purified by silicagel chromatography. 2-(3,4,5-Trimethoxyphenyl)-5-(2-morpholinoethyl)-2,3-dihydro-1,5-benzothiazepine-3,4(5H)-dione (0.25 g) was obtained as oil.

Anal. Calcd. for $C_{24}H_{28}O_6SN_2$: C, 61.01; H, 5.97; N, 5.93.
Found: C, 61.25; H, 6.11; N, 6.08.
IR(Neat)$cm^{-1}$: 3290, 1725, 1665, 1590.
NMR($CDCl_3$)ppm: 2.42 (t, 4H), 2.60 (t, 2H), 3.60 (t, 4H), 3.76—3.88 (m, 9H), 4.02 (t, 2H), 5.96 (s, 1H), 6.52—7.40 (m, 6H).

In a similar manner as described in Example 6, the following compounds were prepared:

2-(4-Chlorophenyl)-5-(2-morpholinoethyl)-2,3-dihydro-1,5-benzothiazepine-3,4(5H)-dione as oil.

Anal. Calcd. for $C_{21}H_{21}O_3SN_2Cl$: C, 60.50; H, 5.08; N, 6.72.
Found: C, 60.61; H, 5.02; N, 6.68.
IR(Neat)$cm^{-1}$: 3300, 1730, 1670, 1585.
NMR($CDCl_3$)ppm: 2.42 (t, m, 4H), 2.58 (t, 2H), 3.58 (t, 4H), 4.12 (t, 2H), 5.54 (s, 1H), 6.82—7.78 (m, 8H).

2-(4-Methylphenyl)-5-(2-morpholinoethyl)-2,3-dihydro-1,5-benzothiazepine-3,4(5H)-dione as oil.

Anal. Calcd. for $C_{22}H_{24}O_3SN_2$: C, 66.65; H, 6.10; N, 7.07.
Found: C, 66.48; H, 6.10; N, 7.10.
IR(Neat)$cm^{-1}$: 3300, 1720, 1680, 1590.
NMR($CDCl_3$)ppm: 2.26 (s, 3H), 2.48 (t, 4H), 2.59 (t, 2H), 3.56 (t, 4H), 4.05 (m, 2H), 5.80 (s, 1H), 6.90—7.68 (m, 8H).

2-(4-Methoxyphenyl)-5-(2-morpholinoethyl)-2,3-dihydro-1,5-benzothiazepine-3,4(5H)-dione as oil.

Anal. Calcd. for $C_{22}H_{24}O_4SN_2$: C, 64.06; H, 5.87; N, 6.21.
Found: C, 64.21; H, 5.67; N, 6.00.
IR(Neat)$cm^{-1}$: 3290, 1730, 1660, 1600, 1585.
NMR($CDCl_3$)ppm: 2.42 (t, 4H), 2.56 (t, 2H), 3.58 (t, 4H), 3.72 (s, 3H), 4.10 (t, 2H), 5.40 (s, 1H), 6.72—7.48 (m, 8H).

2-(2,4-Dichlorophenyl)-5-(2-morpholinoethyl)-2,3-dihydro-1,5-benzothiazepine-3,4(5H)-dione as oil.

Anal. Calcd. for $C_{21}H_{20}O_3SN_2Cl_2$: C, 55.88; H, 4.47; N, 6.21.
Found: C, 56.01; H, 4.43; N, 6.11.
IR(Neat)$cm^{-1}$: 3150, 1720, 1660, 1580.
NMR($CDCl_3$)ppm: 2.36 (t, 4H), 2.50 (t, 2H), 3.54 (t, 4H), 4.01 (t, 2H), 6.04 (s, 1H), 6.80—7.70 (m, 7H).

## Example 7

The solution of 2-(2,4-dichlorophenylmethylidene)-4-(2-dimethylaminoethyl)-2H-1,4-benzothiazine-3(4H)-one hydrochloride (1 g) in 10 ml of chloroform was cooled below 0°C and added with trimethylchlorosilane (2 ml), and then added with 30% hydrogen peroxide (0.29 g) for 30 minutes. The solution was stirred for 30 minutes below 0°C,. and added with 1 ml of water. After further stirring for 1 hour at room temperature, 10 ml of water was added to the reaction mixture. The aqueous phase of the mixture was adjusted to a pH within 8—9 and the chloroform layer was separated from the mixture. The remaining aqueous phase was extracted with 10 ml of chloroform. The chloroform solutions were combined, dried and evaporated under reduced pressure. The oily residue was purified by silicagel chromatography to give 2-(2,4-dichlorophenyl)-5-(2-dimethylaminoethyl)-2,3-dihydro-1,5-benzothiazepine-3,4(5H)-dione (1.0 g) as oil.

Anal. Calcd. for $C_{19}H_{18}O_2SN_2Cl_2$: C, 55.75; H, 4.43; N, 6.84.
Found: C, 55.77; H, 4.40; N, 6.78.
IR(Neat)$cm^{-1}$: 3200, 1735, 1655.
NMR($CDCl_3$)ppm: 2.18 (s, 6H), 2.46 (t, 2H), 3.96 (m, 2H), 6.84—7.54 (m, 7H).

## Example 8

Chlorotrimethylsilane (19.5 g) was dropped, while stirring at −5°C for 30 minutes, into a mixture of 30% hydrogen peroxyde and a solution of 2-(4-methoxyphenylmethylidene)-4-(2-dimethylaminoethyl)-2H-1,4-benzothiazine-3(4H)-one (20 g) in dichloromethane (300 ml). After further stirring at −5°C for 30 minutes, 10 ml of water was added and the reaction mixture was stirred at 23°C for 2 hours. The resulting reaction mixture was diluted with 400 ml of saturated sodium chloride solution and the dichloromethane layer was separated, washed with a mixture of saturated sodium chloride solution (400 ml) and water (300 ml), dried over anhydrous magnesium sulfate and evaporated to remove the solvent. The residue was recrystallized from acetone to give 16.3 g of 2-(4-methoxyphenyl)-2,3-dihydro-5-(2-dimethylaminoethyl)-1,5-benzothiazepine-3,4(5H)-dione hydrochloride as colorless powder having a melting point from 198° to 201°C.

Anal. Calcd. for $C_{20}H_{22}N_2O_3S.HCl$: C, 59.04; H, 5.65; N, 6.88.
Found: C, 59.17; H, 5.68; N, 6.91.
IR(Nujol)$cm^{-1}$: 3350, 2670, 1650, 1638, 1600, 1580.
IR(KBr)$cm^{-1}$: 3400, 2670, 1720, 1660, 1610, 1585.
NMR(DMSO-$d_6$)ppm: 2.80 (s, 6H), 3.36 (br, 2H), 3.72 (s, 3H), 4.42 (br, 2H), 6.06 (br, 1H), 6.40—7.90 (m, 8H).

In a similar manner as described in Example 8, the following compounds were obtained:

2-Phenyl-2,3-dihydro-5-methyl-1,5-benzothiazepine-3,4(5H)-dione as oil.

Anal. Calcd. for $C_{16}H_{13}NO_2S$: C, 67.84; H, 4.63; N, 4.95.
Found: C, 67.68; H, 4.69; N, 5.07.
IR(Neat)$cm^{-1}$: 3300 (br, weak), 1720, 1660, 1580.
NMR($CDCl_3$)ppm: 3.45 (s, 3H), 5.50 (s, 1H), 7.05—7.60 (m, 9H).

2-(4-Chlorophenyl)-2,3-dihydro-5-methyl-1,5-benzothiazepine-3,4-(5H)-dione as oil.

Anal. Calcd. for $C_{16}H_{12}NO_2SCl$: C, 60.47; H, 3.77; N, 4.40.
Found: C, 60.78; H, 3.51; N, 4.26.
IR(Neat)$cm^{-1}$: 1675, 1580.
NMR($CDCl_3$)ppm: 3.53 (s, 3H), 5.12 (s, 1H), 6.95—7.80 (m, 8H).

2-(4-Methoxyphenyl)-2,3-dihydro-5-methoxymethyl-1,5-benzothiazepine-3,4-(5H)-dione, m.p. 124.5° to 125.5°C (recrystallized from ethanol).

Anal. Calcd. for $C_{18}H_{17}NO_4S$: C, 62.97; H, 4.99; N, 4.08.
Found: C, 62.75; H, 4.93; N, 4.06.
IR(Nujol)$cm^{-1}$: 1720, 1670, 1610, 1585.
NMR($CDCl_3$)ppm: 3.54 (s, 3H), 5.06 (s, 1H), 5.42 (q, 2H), 6.76—7.80 (m, 8H).

2-(4-Methoxyphenyl)-2,3-dihydro-5-(2-hydroxyethyl)-1,5-benzothiazepine-3,4(5H)-dione as oil.

Anal. Calcd. for $C_{18}H_{17}NO_{14}S$: C, 62.97; H, 4.99; N, 4.08.
Found: C, 63.14; H, 5.02; N, 3.97.
IR(Neat)$cm^{-1}$: 3425, 1725, 1660, 1610, 1585.

NMR($CDCl_3$)ppm: 3.18 (broad s, 1H), 3.80 (s, 3H), 3.88—4.20 (m, 4H), 5.50 (broad s, 1H), 6.80—7.85 (m, 8H).

2-(4-Methoxyphenyl)-2,3-dihydro-5-(2-chloroethyl)-1,5-benzothiazepine-3,4(5H)-dione as oil.
Anal. Calcd. for $C_{18}H_{16}NO_3SCl$: C, 59.75; H, 4.42; N, 3.87.
Found: C, 60.02; H, 4.38; N, 3.77.
IR(Neat)$cm^{-1}$: 3350, 1725, 1660, 1600, 1580.
NMR($CDCl_3$)ppm: 3.71 (s, 3H), 4.10—4.60 (m, 4H), 5.44 (broad s, 1H), 6.76—8.46 (m, 8H).

2-(4-Methoxyphenyl)-2,3-dihydro-5-(2-bromoethyl)-1,5-benzothiazepine-3,4(5H)-dione as oil.

Anal. Calcd. for $C_{18}H_{16}NO_3SBr$: C, 53.20; H, 3.94; N, 3.44.
Found: C, 53.47; H, 4.01; N, 3.50.
IR(Neat)$cm^{-1}$: 3350, 1720, 1660, 1600, 1580.
NMR($CDCl_3$)ppm: 3.56 (m, 2H), 3.76 (s, 3H), 4.28 (m, 2H), 5.42 (broad s, 1H), 6.76—8.80 (m, 8H).

**Claims**

1. A benzothiazepine derivative of the formula:

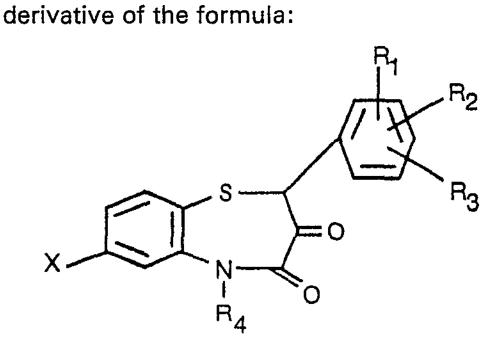

wherein $R_1$, $R_2$ and $R_3$ each denotes hydrogen, a halogen a $C_1$—$C_5$ alkyl group, a $C_1$—$C_5$ alkoxy group or a hydroxy group; $R_4$ denotes a $C_1$—$C_5$ alkyl group, an allyl group, a $C_1$—$C_5$ alkoxy $C_1$—$C_5$ alkyl group, a $C_1$—$C_5$ alkyl group substituted with a hydroxy group or a halogen, a mono- or di-$C_1$—$C_5$ alkylamino $C_1$—$C_5$ alkyl

group or a morpholine $C_1$—$C_5$ alkyl group; X denotes a halogen or, except when $R_4$ is alkyl, hydrogen, or an acid salt thereof.

2. A compound as claimed in Claim 1 wherein $R_1$, $R_2$ and $R_3$ each is hydrogen, chlorine, or methyl or methoxy group; $R_4$ is methyl, methoxymethyl, hydroxyethyl, chloroethyl, bromoethyl, dimethylaminoethyl, or morpholinoethyl group; X is chlorine or, except when $R_4$ is methyl, hydrogen.

3. A compound as claimed in Claim 2 wherein at least one of $R_1$, $R_2$ and $R_3$ is methyl or methoxy group or chlorine and the remainder, if any, is hydrogen.

4. A compound as claimed in Claim 3, wherein $R_1$ is methyl group, methoxy group or chlorine substituted on 4-position of the phenyl group, and $R_2$ and $R_3$ each is hydrogen.

5. A compound as claimed in Claim 4, wherein $R_4$ is dimethylaminoethyl or morpholinoethyl group.

6. 2-(4-Methoxyphenyl)-2,3-dihydro-5-(2-dimethylaminoethyl)-1,5-benzothiazepine-3,4(5H)-dione, or an acid salt thereof.

7. A benzothiazepine derivative of the formula:

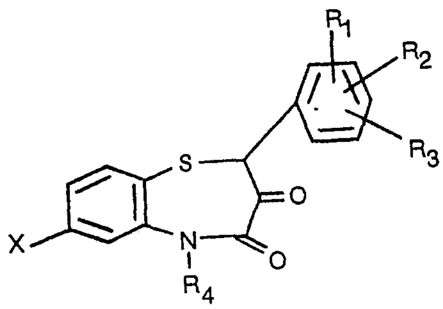

wherein $R_1$, $R_2$ and $R_3$ each denotes hydrogen, a halogen, a $C_1$—$C_5$ alkyl group, a $C_1$—$C_5$ alkoxy group or a hydroxy group; $R_4$ denotes a $C_1$—$C_5$ alkyl group, an allyl group, a $C_1$—$C_5$ alkoxy $C_1$—$C_5$ alkyl group, a $C_1$—$C_5$ alkyl group substituted with a hydroxy group or a halogen, a mono- or di-$C_1$—$C_5$ alkylamino $C_1$—$C_5$ alkyl group or a morpholine $C_1$—$C_5$ alkyl group; X denotes a halogen or hydrogen, or an acid salt thereof, for use in the treatment of the human or animal body by therapy.

8. A compound as claimed in Claim 7 wherein $R_1$, $R_2$ and $R_3$ each is hydrogen, chlorine, or methyl or methoxy group; $R_4$ is methyl, methoxymethyl, hydroxyethyl, chloroethyl, bromoethyl, dimethylaminoethyl, or morpholinoethyl group; X is chlorine or hydrogen.

9. A compound as claimed in Claim 8 wherein at least one of $R_1$, $R_2$ and $R_3$ is methyl or methoxy group or chlorine and the remainder, if any, is hydrogen.

10. A compound as claimed in Claim 9, wherein $R_1$ is methyl group, methoxy group or chlorine substituted on 4-position of the phenyl group, and $R_2$ and $R_3$ each is hydrogen.

11. A pharmaceutical composition comprising a compound as defined in any one of Claims 1 to 10 in admixture with a pharmacologically acceptable excipient.

12. The use of a compound as defined in any one of Claims 1 to 10 in the manufacture of a medicament for analgesic, antipyretic or antiarrhythmic use.

**Patantansprüche**

1. Benzothiazepinderivate der Formel:

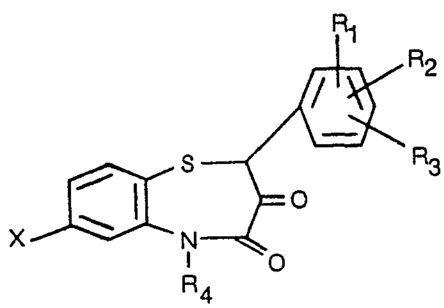

worin $R_1$, $R_2$ und $R_3$ jeweils Wasserstoff, ein Halogen, eine $C_1$—$C_5$-Alkylgruppe, eine $C_1$—$C_5$-Alkoxygruppe oder eine Hydroxygruppe bedeuten; $R_4$ eine $C_1$—$C_5$-Alkylgruppe, eine Allylgruppe, eine $C_1$—$C_5$-Alkoxy-$C_1$—$C_5$-alkylgruppe, eine $C_1$—$C_5$-Alkylgruppe, die mit einer Hyhdroxygruppe oder einem Halogen substituiert ist, eine mono- oder di-$C_1$—$C_5$-Alkylamino-$C_1$—$C_5$-alkylgruppe oder eine Morpholin-$C_1$—$C_5$-Alkylgruppe bedeutet; X ein Halogen oder, ausgenommen $R_4$ steht für Alkyl, Wasserstoff bedeutet oder ein Säuresalz davon.

2. Verbindungen nach Anspruch 1, worin $R_1$, $R_2$ und $R_3$ jeweils Wasserstoff, Chlor oder eine Methyl- oder Methoxygruppe bedeuten; $R_4$ eine Methyl-, Methoxymethyl-, Hydroxyäthyl-, Chloräthyl-, Bromäthyl-, Dimethylaminoäthyl- oder eine Morpholinoäthylgruppe bedeutet; und X Chlor oder, ausgenommen $R_4$ steht für Alkyl, Wasserstoff bedeutet.

3. Verbindungen nach Anspruch 2, worin mindestens einer der Reste $R_1$, $R_2$ oder $R_3$ eine Methyl- oder Methoxygruppe oder Chlor bedeutet und die restlichen Substituenten, falls vorhanden, Wasserstoff bedeuten.

4. Verbindungen nach Anspruch 3, worin $R_1$ eine Methylgruppe, eine Methoxygruppe oder Chlor in 4-Stellung der Phenylgruppe bedeutet und $R_2$ und $R_3$ jeweils Wasserstoff sind.

5. Verbindungen nach Anspruch 4, worin $R_4$ eine Dimethylaminoäthyl- oder Morpholinoäthylgruppe bedeutet.

6. 2-(4-Methoxyphenyl)-2,3-dihydro-5-(2-dimethylaminoäthyl)-1,5-benzothiazepin-3,4(5H)-dion oder ein Säuresalz davon.

7. Benzothiazepinderivate der Formel:

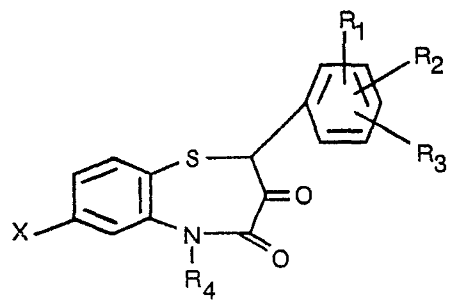

worin $R_1$, $R_2$ und $R_3$ jeweils Wasserstoff, ein Halogen, eine $C_1$—$C_5$-Alkylgruppe, eine $C_1$—$C_5$-Alkoxygruppe oder eine Hydroxygruppe bedeuten; $R_4$ eine $C_1$—$C_5$-Alkylgruppe, eine Allylgruppe, eine $C_1$—$C_5$-Alkoxy-$C_1$—$C_5$-alkylgruppe, eine $C_1$—$C_5$-Alkylgruppe, die mit einer Hydroxygruppe oder einem Halogen substituiert ist, eine mono- oder di-$C_1$—$C_5$-Alkylamino-$C_1$—$C_5$-alkylgruppe oder eine Morpholin-$C_1$—$C_5$-Alkylgruppe bedeutet; X ein Halogen oder Wasserstoff bedeutet, oder ein Säureadditionssalz davon, zur Anwendung bei der therapeutischen Behandlung von Mensch und Tier.

8. Verbindungen nach Anspruch 7, worin $R_1$, $R_2$ und $R_3$ jeweils Wasserstoff, Chlor oder eine Methyl- oder Methoxygruppe bedeuten; $R_4$ eine Methyl-, Methoxymethyl-, Hydroxyäthyl-, Chloräthyl-, Bromäthyl-, Dimethylaminoäthyl- oder eine Morpholinoäthylgruppe bedeutet; X Chlor oder Wasserstoff bedeutet.

9. Verbindungen nach Anspruch 8, worin mindestens einer der Reste $R_1$, $R_2$ und $R_3$ Methyl- oder Methoxygruppe oder Chlor bedeutet und die restlichen Substituenten, falls vorhanden, Wasserstoff bedeuten.

10. Verbindungen nach Anspruch 9, worin $R_1$ eine Methyl- oder Methoxygruppe oder Chlor in 4-Stellung der Phenylgruppe bedeutet und $R_2$ und $R_3$ jeweils Wasserstoff sind.

11. Pharmazeutisches Mittel, welches eine Verbindung nach einem der Ansprüche 1 bis 10 zusammen mit einem pharmakologisch verträglichen Exzipienten enthält.

12. Verwendung einer Verbindung nach einem der Ansprüche 1 bis 10 zur Herstellung eines Analgetikums, Antipyretikums oder Antiarrhythmikums.

**Revendications**

1. Dérivé de benzothioazepine ayantg la formule

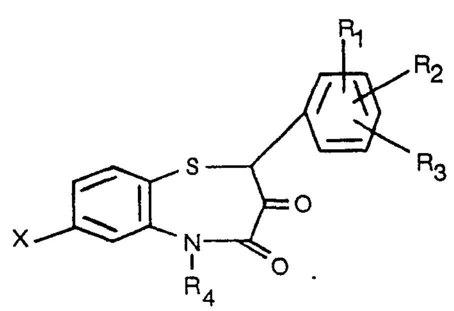

dans laquelle $R_1$, $R_2$ et $R_3$ signifient chacun de l'hydrogène, un halogène, un groupe alkyle en $C_1$ à $C_5$, un groupe alcoxy en $C_1$ à $C_5$ ou un groupe hydroxy;

$R_4$ représente un groupe alkyle en $C_1$ à $C_5$, un groupe allyle, un groupe (alcoxy en $C_1$ à $C_5$)-alkyle en $C_1$ à $C_5$, un groupe alkyle en $C_1$ à $C_5$ substitué par un groupe hydroxy ou un halogène, un groupe mono- ou di-

(alkylamino en $C_1$ à $C_5$)-alkyle en $C_1$ à $C_5$ ou un groupe (morpholino)-alkyle en $C_1$ à $C_5$; X représente un halogène ou bien, lorsque $R_4$ n'est pas alkyle, de l'hydrogène, ou un sel d'acide de ce composé.

2. Composé selon la revendication 1 dans lequel $R_1$, $R_2$ et $R_3$ sont de l'hydrogène, du chlore, du méthyle ou du méthoxy; $R_4$ représente méthyle, méthoxyméthyle, hydroxyéthyle, chloréthyle, brométhyle, diméthylaminoéthyle ou morpholinoéthyle et X représente du chlore ou de l'hydrogène mais seulement lorsque $R_4$ n'est pas méthyle.

3. Composé selon la revendication 2 dans lequel au moins un des symboles $R_1$, $R_2$ et $R_3$ représente méthyle ou méthoxy ou du chlore, et le symbole éventuellement restant signifie de l'hydrogène.

4. Composé selon la revendication 3 dans lequel $R_1$ est un groupe méthyle, methoxy cu chlore en position 4 de l'anneau phényle, et $R_2$ et $R_3$ sont chacun de l'hydrogène.

5. Composé selon la revendication 4 dans lequel $R_4$ est un groupe diméthylaminoéthyle ou morpholinoéthyle.

6. 2-(4-Methoxyphényl)-2,3-dihydro-5-(2-diméthylaminoéthyl)-1,5-benzodiazépin-3,4(5H)-dione ou un de ses sels d'acide.

7. Dérivé de benzothiazépine de formule

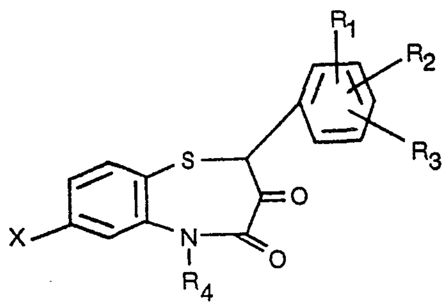

dans laquelle $R_1$, $R_2$ et $R_3$ signifient chacun de l'hydrogène, un halogène, un groupe alkyle en $C_1$ à $C_5$, un groupe alcoxy en $C_1$ à $C_5$ ou un groupe hydroxy; $R_4$ représente un groupe alkyle en $C_1$ à $C_5$, un groupe allyle, un groupe (alcoxy en $C_1$ à $C_5$)-alkyle en $C_1$ à $C_5$, un groupe alkyle en $C_1$ à $C_5$ substitué par un groupe hydroxy ou un halogène, un groupe mono- ou di-(alkylamino en $C_1$ à $C_5$)-alkyle en $C_1$ à $C_5$ ou un groupe (morpholino)-alkyl en $C_1$ à $C_5$; X représente un halogène ou de l'hydrogène, ou un de ses sels d'acide, pour l'utilisation dans le traitement du corps humain ou animal par une thérapie.

8. Composé selon la revendication 7 dans lequel $R_1$, $R_2$ et $R_3$ sont de l'hydrogène, du chlore, du méthyle ou du méthoxy; $R_4$ représente méthyle, méthoxyméthyle, hydroxyéthyle, chloréthyle, brométhyle, diméthylaminoéthyle ou morpholinoéthyle et X représente du chlore ou de l'hydrogène.

9. Composé selon la revendication 8 dans lequel au moins un des symboles $R_1$, $R_2$ et $R_3$ représente méthyle ou méthoxy ou du chlore, et le symbole éventuellement restant signifie de l'hydrogène.

10. Composé selon la revendication 9 dans lequel $R_1$ est un groupe méthyle, methoxy ou chlore en position 4 de l'anneau phényle, et $R_2$ et $R_3$ sont chacun de l'hydrogène.

11. Composition pharmaceutiquement comprenant un composé selon l'une quelconque des revendications 1 à 10 en mélange avec un excipient acceptable du point de vue pharmacologique.

12. Utilisation d'un composé selon l'une quelconque des revendications 1 à 10, dans la fabrication d'un médicament ayant une utilité analgésique, antipyrétique ou antiarythmique.